# EUROPÄISCHE PATENTSCHRIFT

(11) **EP 1 196 780 B1**
(45) Veröffentlichungstag und Bekanntmachung des Hinweises auf die Patenterteilung: **06.10.2004**
(21) Anmeldenummer: 00949367.7
(22) Anmeldetag: 17.07.2000
(51) Int. Cl.: G01N 33/58, G01N 33/96

(54) **HERSTELLUNG UND ANWENDUNG VON LUMINESZIERENDEN MIKRO- UND NANOPARTIKELN**
PRODUCTION AND USE OF LUMINESCENT MICROPARTICLES AND NANOPARTICLES
PRODUCTION ET UTILISATION DE MICROPARTICULES ET DE NANOPARTICULES LUMINESCENTES

(30) Priorität: 15.07.1999 DE 19933104
(43) Veröffentlichungstag der Anmeldung: 17.04.2002
(73) Patentinhaber: Presens Precision Sensing GmbH, 93053 Regensburg (DE)
(72) Erfinder: KLIMANT, Ingo, D-93051 Regensburg (DE)
(74) Vertreter: Weiss, Wolfgang, Dipl.-Chem. Dr.
(86) Internationale Anmeldenummer: PCT/EP2000/006832
(87) Internationale Veröffentlichungsnummer: WO 2001/006227

(56) Entgegenhaltungen:
- WO-A-95/14928
- WO-A-96/21154
- WO-A-99/06821
- GB-A- 2 132 348
- PATENT ABSTRACTS OF JAPAN vol. 011, no. 379 (C-463), 10. Dezember 1987 (1987-12-10) & JP 62 148580 A (TOAGOSEI CHEM IND CO LTD;OTHERS: 01), 2. Juli 1987 (1987-07-02)
- HUBER, CHRISTIAN ET AL: "Optical sensor for seawater salinity" FRESENIUS' J. ANAL. CHEM. (2000), 368(2-3), 196-202 , XP000974975
- LIEBSCH, GREGOR ET AL: "Luminescence lifetime temperature sensing based on sol -gels and poly ( acrylonitrile )s dyed with ruthenium metal-ligand complexes" ADV. MATER. (WEINHEIM, GER.) (1999), 11(15), 1296-1299 , XP000971460

## Beschreibung

Die Erfindung betrifft die Zusammensetzung, Herstellung und Verwendung von lumineszierenden Mikro- und Nanopartikeln mit langlebiger Lumineszenz. Diese Partikel können entweder als interne Standards zur Referenzierung von Fluoreszenz- oder Phosphoreszenzsignalen (Lumineszenzsignalen) oder als Marker zur Markierung und Detektion von Biomolekülen verwendet werden. Langlebige Luminenzfarbstoffe werden in inerter Form in feste Materialien eingebaut, das heißt vom Einfluß chemischer und biologischer Substanzen in gasförmigen und wässrigen Proben abgeschirmt. In dieser eingebauten Form bleiben die photophysikalischen Eigenschaften der Farbstoffe (spektrale Charakteristik, Lumineszenzabklingzeit und Lumineszenzanisotropie) von wechselnden Probenparametern unbeeinflußt.

Als Einbaumatrix werden insbesondere dichte anorganische Materialien oder organische Polymere ausgewählt, die aufgrund ihrer Struktur die Aufnahme von Biomolekülen kleinen neutralen Molekülen sowie ionischen Substanzen ausschließen. insbesondere der störende Einfluß von molekularem Sauerstoff, einem effizienten Fluoreszenz- oder Phosphoreszenzlöscher, auf die Lumineszenzmessungen wird auf diese Weise ausgeschlossen bzw. stark reduziert. Die Oberfläche der Nano- und Mikropartikel kann mit reaktiven chemischen Gruppen versehen sein, um die kovalente Kopplung von Biomolekülen oder/und lumineszierenden Indikator-Farbstoffen zu ermöglichen. Ferner kann die Oberfläche mit chemischen Gruppen versehen sein, um das Aggregieren der Partikel zu verhindern.

Die Messung der Lumineszenz ist eine weit verbreitete Methode in der Biound Chemoanalytik. Ihre Attraktivität verdankt sie ihrer hohen Empfindlichkeit, der Vielseitigkeit sowie der Eliminierung der Strahlenbelastung durch radioaktive Markierungsreagenzien. In der Praxis werden in der Regel Lumineszenzmarker, die sich durch eine hohe Quantenausbeute auszeichnen, eingesetzt. Meist wird die Lumineszenzintensität des Lumineszenzenzmarkers mit dem zu bestimmenden Parameter der Probe korreliert. Nachteilig wirkt sich bei solchen Bestimmungsmethoden aus, daß die quantitative Auswertung der Lumineszenzintensität durch eine Vielzahl von Faktoren gestört wird. Dabei kann es sich zum einen um Schwankungen im optischen System (Strahlungsintensität der Lichtquelle, Empfindlichkeit des Detektors und Transmission des optischen Weges) aber auch um intrinsische optische Eigenschaften der Probe (Färbung oder Trübung) handeln.

Um diese Störeinflüsse zu eliminieren bzw. zu vermindern, benötigt man geeignete Methoden zur Referenzierung der Lumineszenzsignate. Eine in WO99/O6821 (Klimant) beschriebene Methode zum Referenzieren von Lumineszenzsignalen beruht darauf, daß zur Probe ein lumineszierender Referenzfarbstoff zugegeben wird, der ähnliche (im besten Fall identische) spektrale Eigenschaften wie der eigentliche Lumineszenzmarker aufweist. In Kombination mit einer Frequenzrnodulations- oder zeitaufgelösten Lumineszenzmessung wird auf diese Weise die Intensitätsinformation in ein Phasensignal oder einen zeitabhängigen Parameter umgewandelt. Um auf diese Weise eine fehlerfreie Referenzierung des Meßsignals zu realisieren, werden inerte lumineszierende Referenzstandards benötigt, deren Lumineszenzeigenschaften von den Probenparametern nicht beeinflußt werden. Dafür kommen zum Beispiel phosphoreszierende anorganische Feststoffe wie zum Beispiel mit Cr(III) dotierte Mischoxide in Frage, die in gepulverter Form der Probe zugemischt werden können. Andererseits können hierzu auch langlebige Lumineszenzfarbstoffe in Träger aus organischen oder anorganischen Materialien eingebaut und der Probe zugemischt werden.

Eine weitere Art der Störung der quantitativen Auswertung von Fluoreszenzintensitätssignalen ist das Auftreten von Eigenfluoreszenz in der Probe. Insbesondere natürliche Proben wie Blut oder Serum können eine Vielzahl an fluoreszierenden Substanzen aufweisen. Ist die Signal intensität des fluorimetrischen Assays sehr gering, kann durch Eigenfluoreszenz die Messung sogar unmöglich sein. Ein verbreitete Methode um das eigentliche Lumineszenzsignal vom unspezifischen Untergrundsignal abzutrennen besteht darin, langlebig emittierende Lumineszenzfarbstoffe als Marker zu verwenden. Mit Hilfe zeitaufgelöster Lumineszenztechniken ist es möglich, das verzögerte Meßsignal zeitlich von der kurzlebigen Untergrundfluoreszenz zu trennen. Für diese Methode werden hauptsächlich phosphoreszierende Chelate der Seltenerdenmetalle (insbesondere die des Europium oder Terbium) eingesetzt. Diese Farbstoffe besitzen aber den Nachteil, dass sie nur mit UV-Lichtquellen angeregt werden können. Außerdem sind die verwendeten Chelate in gelöster Form in wässrigen Systemen häufig instabil, d.h. es kommt zu Ligandenverlust. Als langlebige Marker kommen potentiell aber auch lumineszierende Metall-ligand-Komplexe, insbesondere mit Ruthenium(II) als Zentralatom in Frage. Werden diese Farbstoffe in gelöster Form wässrigen Systemen zugegeben, wird deren Lumineszenz in der Regel durch molekularen Sauerstoff, starke Oxidationsmittel oder Reduktionsmittel gelöscht.

Weiterhin können auch Lumineszenzindikatoren, beispielsweise zur Bestimmung des pH-Werts, der Konzentration bzw. Aktivität von lonen oder kleinen Molekülen, eingesetzt werden, deren Lumineszenzintensität durch direkte oder indirekte Wechselwirkung mit dem zu bestimmenden Parameter, z.B. durch Reaktion mit einem Analyten oder als Transducer, von der Konzentration bzw. Aktivität des zu bestimmenden Parameters, z.B. eines Analyten oder des pH-Werts, abhängt.

Für alle genannten Methoden ist es unbedingt notwendig, daß die photophysikalischen Eigenschaften des Lumineszenzfarbstoffs nicht von den Probenparametern beeinflußt werden. Werden solche Farbstoffe der Probe im gelösten Zustand zugegeben oder mit der Probe in zumindest indirekten Kontakt gebracht, sind diese Voraussetzungen nicht gegeben. Insbesondere Fluoreszenz- oder Phosphoreszenzlöschungen durch molekularen Sauerstoff sowie oxidative und reduktive Löscher bewirken Fehlinterpretationen des Meßsignals.

Um inerte langlebige Lumineszenzmarker und Lumineszenzfarbstoffe zur Referenzierung der Lumineszenzintensität von Lumineszenzindikatoren zur Verfügung zu haben, müssen die Lumineszenzfarbstoffe in feste Materialien eingebaut werden, damit sie mit der Probe nicht in Wechselwirkung treten können.

Die vorliegende Anmeldung beschreibt sowohl neue lumineszierende Mikround Nanopartikel, deren lumineszenze Eigenschaften nicht oder nur gering von der Zusammensetzung der Probe abhängen, als auch Verfahren zu deren Herstellung. Es werden außerdem Anwendungsmöglichkeiten der in Form von Nano- und Mikropartikeln vorliegenden Lumineszenzmarker bzw. Lumineszenzfarstoffe zur Referenzierung der Lumineszenzintensität von Lumineszenzindikatoren beschrieben.

Ein Gegenstand der Anmeldung sind daher lumineszierende, insbesondere phosphoreszierende Mikro- und Nanopartikel, die lumineszierende Substanzen, z.B. Metall-Liganden-Komplexe mit langen Lumineszenzabklingzeiten in einer festen Matrix enthalten, so dass sie gegenüber chemischen Parametern der Umgebung, z.B. einer Probe, abgeschirmt sind und deren Lumineszenzeigenschaften wie etwa Quantenausbeute, spektrale Eigenschaften, Lumineszenzabklingzeit oder/und Anisotropie von der jeweiligen Umgebung, z.B. der jeweiligen Probenzusammensetzung, im Wesentlichen unabhängig sind. "Unabhängig" im Sinne der gegenständlichen Anmeldung bedeutet, dass die Abhängigkeit der Lumineszenzabklingzeit und ggf. weiterer Lumineszenzeigenschaften vom pO₂ und ggf. anderen störenden Substanzen der Umgebung der in den erfindungsgemäßen Partikeln vorliegenden Lumineszenzfarbstoffe, weiche sich in zumindest in indirektem Kontakt mit der Probe befinden, geringer ist als die Abhängigkeit der Lumineszenzabklingzeit und ggf. weiteren Lumineszenzeigenschaften der entsprechenden Farbstoffe, welche sich, ohne die erfindungsgemäße Abschirmung, in zumindest indirektem Kontakt mit der Probe befinden. Vorzugsweise ist die Lumineszenzabklingzeit der in den erfindungsgemäßen Partikeln vorliegenden Lumineszenzfarbstoffe in luftgesättigter Umgebung um höchstens 20%, besonders bevorzugt um höchstens 15% und am meisten bevorzugt um höchstens 10% geringer als in O₂-freier Umgebung, jeweils bei Raumtemperatur. Ohne Abschirmung wird hingegen eine Abnahme der Lumineszenzabklingzeit um deutlich mehr als 80% in luftgesättigter Umgebung gegenüber einer O₂-freien Umgebung gefunden.

Die lumineszierenden Metall-Liganden-Komplexe sind vorzugsweise Verbindungen von Übergangsmetallen wie Ruthenium(II), Osmium(II), Rhenium(I), Iridium(III), Platin(II) und Palladium(II) als Zentralatom. Die Komplexliganden werden vorzugsweise aus zwei- oder/und dreizähnigen Liganden mit N-Heterozyklen, beispielsweise Polypyridyl-Liganden wie etwa 2,2'-Bipyridin, Bipyrazin, Phenanthrolin, Terpyridil oder deren Abkömmlingen ausgewählt. Besonders bevorzugte Beispiele von Metall-Liganden-Komplexen sind die Triskomplexe von Ruthenium(II) mit 2,2'-Bipyridyl, 1,10-phenanthrolin, 4,4-Diphenyl-2,2'-bipyridyl und 4,7-Diphenyl-1,10-Phenanthrolin als Liganden. Weiterhin besonders bevorzugt sind Carbonylkomplexe von Re(I) mit zusätzlichen Poly-N-heterozyklischen Liganden wie etwa 2,2'-Bipyridyl und 1,10- phenanthrolin oder Abkömmlingen davon. Ebenfalls bevorzugt sind als Metall-Liganden-Komplexe die Porphyrinkomplexe von Pt(II) oder Pd(II) als Zentralatom, die sich durch intensive Phosphoreszenz bei Raumtemperatur auszeichnen. Die Lumineszenzabklingzeiten der Verbindungen betragen vorzugsweise ≥ 100 Nanosekunden, besonders bevorzugt ≥ 400 Nanosekunden. Erfindungsgemäß können auch Seltenerdmetalle wie beispielsweise die Lanthaniden Tb(III) oder Eu(III) oder andere Substanzen als langlebige Lumineszenzfarbstoffe eingesetzt werden.

Die lumineszierenden Mikro- und Nanopartikel haben vorzugsweise eine mittlere Größe im Bereich von 20 nm bis 10 *µ*m, besonders bevorzugt von 50 nm bis 1 *µ*m. Die lumineszierenden Verbindungen werden in Materialien eingebaut, die sich durch geringe Permeabilität (d.h. geringe Diffusionskonstanten und geringe Löslichkeit) für Wasser, löschende gasförmige Substanzen (z.B. O₂) und Störsubstanzen auszeichnen. Beispiele für geeignete Materialien sind nichtporöse Gläser, insbesondere Gläser, die nach einem Sol-Gel-Verfahren beispielsweise aus Silizium-, Titan-, Zirkonium- oder Zinn-enthaltenden Verbindungen, z.B. Alkoholaten wie etwa Zinntetraalkoholaten hergestellt wurden.

Die Herstellung solcher Gläser nach Standardmethoden führt zu Materialien, die durch eine mikroporöse Struktur gekennzeichnet sind. Eingebaute Lumineszenzfarbstoffe sind damit für gelöste Probenbestandteile und insbesondere Sauerstoff zugänglich und können damit gelöscht werden. Die in dieser Erfindung beschriebenen Sol-Gläser werden aus diesem Grund durch Erhitzen auf eine erhöhte Temperatur von z.B. 200°C in einem besonderen Schritt der Herstellung verdichtet. Nach der Hydrolyse des Sol-Gel-Precursors, z.B. Tetramethoxysilan, wird unter Vakuum das Lösungsmittel abgezogen und das Sol-Gel noch vor der endgültigen Vernetzung getrocknet. Auf diese Weise entsteht eine dichte nichtporöse Glasmatrix. Biomoleküle sowie chemische Verbindungen können in diese dichte Matrix nicht eindringen und beeinflussen damit nicht die Lumineszenzeigenschaften der eingebauten Farbstoffe. Es wurden inerte phosphoreszierende Sol-Gel Gläser mit den Farbstoffen Ruthenium(II)-tris-1,10-phenanthrolin sowie Ruthenium(II)-tris-4,7-diphenyl-1,10-phenanthrolin mit Farbstoffgehalten bis zu 40 mM (bezogen auf kg SiO₂) nach diesem Verfahren hergestellt. Diese Materialien zeichnen sich durch intensive Raumtemperaturlumineszenz aus, die durch Sauerstoff nicht gelöscht wird. Da im Herstellungsprozess die Sol-Gel-Phosphore entweder in monolithischer Form oder als dünne Filme entstehen, müssen Mikropartikel durch Pulverisieren hergestellt werden. Anschließende Silanisierung der Partikel führt zu reaktiven Oberflächen, die zum kovalenten Koppeln von Lumineszenzindikatoren oder Biomolekülen genutzt werden können. Die Oberfläche der Partikel kann hierzu beispielsweise mit Amino-, Epoxy-, Hydroxyl-, Thiol- oder/und Carboxylgruppen versehen werden.

Eine Alternative zur Herstellung von inerten Lumineszenzpartikeln besteht darin, organische Polymere als Einbettungsmatrix zu verwenden, die sich zum einen durch eine sehr geringe Gaspermeabilität (zum Ausschluss von Sauerstoff) und zum anderen durch eine minimale Wasseraufnahme (um das Eindringen ionischer Verbindungen zu verhindern) auszeichnen. Geeignete Polymere sind Polyvinylchlorid, Polyvinylidenchlorid, Poly(meth)acrylpolymere und insbesondere Polyacrylnitril sowie Copolymere davon.

Polyacrylnitril (PAN) besitzt eine extrem geringe Gaspermeabilität, teilweise hydrophile Eigenschaften und eine sehr geringe Aufnahmekapazität für Wasser (ca. 2%). Außerdem können die an der Oberfläche befindlichen Nitrilgruppen der Polymerpartikel beispielsweise zu Carboxylgruppen oder/und Amidgruppen verseift bzw. zu Amingruppen umgesetzt werden, die dann für die kovalente Bindung von diversen Biomolekülen zur Verfügung stehen. Aus diesem Grund ist Polyacrylnitril die optimale Einbettungsmatrix für Lumineszenzfarbstoffe als Basis für inerte Nano- und Mikropartikel.

Weiterhin können auch Polyacrylnitril-Copolymere oder Mischpolymere mit Polyacrylnitril eingesetzt werden, d.h. Polymere, die Acrylnitril und zusätzlich ein oder mehrere Monomere enthalten, insbesondere Polyacrylnitril-Co- oder Mischpolymere mit einem PAN-Gewichtsanteil von mindestens 50%, vorzugsweise mindestens 70% und besonders bevorzugt mindestens 90%. Ein Copolymer enthält PAN und ein Comonomer in einer Polymerkette. Ein Mischpolymer enthält eine PAN bzw. PAN-Copolymerkomponente in einer Polymerkette und mindestens eine Nicht-PAN-Kompenente in einer anderen Polymerkette. Geeignete zusätzliche Monomere für Copolymere und Mischpolymere sind Monomere mit hydrophilen oder/und reaktiven Gruppen z.B. Acrylsäure, Acrylamine und Acrylester, z.B. Polyethylenglykol-Acrylester oder Gemische davon. Dabei können sich die hydrophilen Gruppen bevorzugt auf der Partikeloberfläche anreichern. Die an der Oberfläche befindlichen hydrophilen oder/und reaktiven Gruppen können dann zur Kopplung von Bindepartnern wie Biomolekülen oder lumineszierenden Indikatormolekülen verwendet werden. Weiterhin können diese Gruppen auch zur Vermeidung der Aggregation von Partikeln beitragen.

Die Herstellung lumineszierender Mikro- und Nanopartikel auf der Basis von Polyacrylnitril (PAN) kann auf verschiedenen Wegen erfolgen.
A. Ausfällung der Partikel aus einer Lösung von PAN bzw. einem PAN-Copolymer oder Mischpolymer in einem organischen Lösungsmittel(gemisch), z.B. Dimethylformamid durch kontrolliertes Zutropfen von Wasser, wässrigen Lösungen, z.B. einer NaCI-Lösung, oder anderen Flüssigkeiten, die mit dem Polymerlösungsmittel mischbar sind, jedoch eine Verringerung der Löslichkeit und somit eine Ausfällung des Polymers mit dem Lumineszenzfarbstoff bewirken. Die Polymerlösung enthält gleichzeitig den gelösten Lumineszenzfarbstoff. Diese Verfahrensvariante ist besonders einfach und daher bevorzugt.
B. Ausfällung der Partikel aus einer Lösung von PAN bzw. einem PAN-Copolymer oder Mischpolymer in einem organischen Lösungsmittel(gemisch), z.B. Dimethylformamid, durch kontrolliertes Zutropfen von Wasser, wässrigen Lösungen, z.B. einer NaCl-Lösung, oder anderen Flüssigkeiten, die mit dem Polymerlösungsmittel mischbar sind, jedoch eine Ausfällung des Polymers bewirken. Die Polymerlösung enthält keinen gelösten Lumineszenzfarbstoff. Der Lumineszenzfarbstoff wird nachträglich durch Diffusion in die Partikel eingetragen.
C. Herstellung der Partikel durch Sprühen einer Lösung von PAN bzw. einem PAN-Copolymer oder Mischpolymer in einem organischen Lösungsmittel(gemisch), z.B. Dimethylformamid, die den Lumineszenzfarbstoff z.B. in Wasser oder Ethanol enthält, wobei das Lösungsmittel verdunstet.

In allen Vorschriften kann der Durchmesser der Partikel durch Veränderung des Anteils an Polymer in der Lösung gezielt eingestellt werden. Mit abnehmendem Anteil an Polymer reduziert sich auch der Durchmesser der Partikel.

Nach der Herstellung und Isolierung der lumineszierenden Mikro- und Nanopartikel kann die Aktivierung der Oberfläche mit reaktiven Carboxylgruppen, z.B. durch Verseifung der oberflächengebundenen Nitrilgruppen in Base, z.B. konzentrierter Natronlauge, erfolgen. Die Carboxylgruppen werden aus zwei Gründen benötigt. Zum einen können so stabile Dispersionen in (pH-)gepufferten Systemen hergestellt werden und zum anderen können Biomoleküle und Lumineszenzindikatoren an der Oberfläche kovalent gebunden werden.

Erfindungsgemäße Partikel, deren Oberfläche durch reaktive Gruppen modifiziert ist, können zur kovalenten Kopplung von Lumineszenzindikatoren oder/und Biomolekülen eingesetzt werden. Bei den Lumineszenzindikatoren kann es sich um ähnliche Verbindungen handeln, wie sie in der Partikelmatrix eingeschlossen sind. Im Unterschied zu den eingeschlossenen lumineszierenden Verbindungen stehen die an die Oberfläche gekoppelten Lumineszenzindikatoren mit der Umgebung in Kontakt, so dass sie auf chemische Umgebungsparameter reagieren können. Derart modifizierte Partikel können als Lumineszenzindikatoren mit interner Referenzierung - eingesetzt werden. Andererseits oder zusätzlich können auch Biomoleküle wie Toxine, Hormone, Hormonrezeptoren, Peptide, Proteine, Lektine, Oligonukleotide, Nukleinsäuren, Antikörper, Antigene, Viren und Bakterien an die Oberfläche der Partikel gekoppelt werden. Die Kopplung erfolgt über bekannte Methoden, z.B. unter Verwendung von bifunktionellen Linkermolekülen.

Außerdem können die Partikel als Standards zur Referenzierung von Lumineszenzintensitätssignalen in fluorometrischen Assays, z.B. bei der diagnostischen Bestimmung von Analyten, eingesetzt werden.

Die Mikro- und Nanopartikel können zum einen als lumineszierende Standards zur Konvertierung der Lumineszenzintensität von an der Oberfläche gebundenen bzw. in der Umgebung befindlichen Lumineszenzindikatoren in Phasensignale oder zeitabhängige Parameter (beispielsweise zur Referenzierung des Lumineszenzintensitätssignals von optischen Lumineszenzsensoren, wobei die Partikel mit einem Luminenszenzindikator in einer festen Phase, wie in WO99/O6821 (Klimant) beschrieben, gemeinsam immobilisiert werden) und zum anderen als Lumineszenzmarker für die hochempfindliche Detektion bzw. Bestimmung von Biomolekülen eingesetzt werden.

Ein Gegenstand der Erfindung ist somit auch ein Verfahren zur lumineszenzoptischen Bestimmung eines biochemischen oder chemischen Parameters unter Verwendung zweierverschiedener Lumineszenzfarbstoffe, die unterschiedliche Abklingzeiten aufweisen und das Zeit- oder Phasenverhalten der sich ergebenden Lumineszenzantwort zur Bildung einer Referenzgröße für die Bestimmung des Parameters verwendet wird, wobei der erste Lumineszenzfarbstoff zumindest in der Lumineszenzintensität auf den Parameter anspricht und der zweite zumindest in der Lumineszenzintensität und der Abklingzeit im Wesentlichen nicht auf den Parameter anspricht, wobei das Verfahren dadurch gekennzeichnet ist, dass der zweite Lumineszenzfarbstoff in Form von erfindungsgemäßen Partikeln eingesetzt wird. Als Referenzgröße wird vorzugsweise ein Verhältnis der beiden Lumineszenzintensitätsanteile verwendet, welches unabhängig von der Gesamtintensität des Lumineszenzsignals ist. Alternativ kann als Referenzgröße auch die Phasenverschiebung der Lumineszenzantwort des ersten Lumineszenzfarbstoffs zu der des zweiten Lumineszenzfarbstoffs verwendet werden. Außerdem kann als Referenzgröße auch die gemessene Phasenverschiebung des Summensignals aus dem Signal des ersten Lumineszenzfarbstoffs und dem verzögerten Referenzsignal des zweiten Lumineszenzfarbstoffs verwendet werden. Für weitere Einzelheiten des Verfahrens und einer zur Durchführung des Verfahrens geeigneten Vorrichtung wird auf WO99/O6821 verwiesen.

Weiterhin soll die Erfindung durch die nachfolgenden Beispiele erläutert werden.

### Beispiele

### Beispiel 1

### Herstellung von lumineszierenden Nanopartikeln aus Polyacrylnitril und [Ruthenium(II)-tris-4,7-diphenyl-1,10 phenanthrolin]²⁺

1 g n-Polyacrylnitril (Polysciences Inc., MW 150000) wird zusammen mit 10mg Ruthenium(II)-tris-4,7-diphenyl-1,10-phenanthrolin-perchlorat in 100 ml Dimethylformamid (DMF) gelöst und in ein 11 Becherglas gefüllt. Zu dieser Lösung werden 400 ml H₂O unter ständigem Rühren langsam zugetropft. Es entsteht eine leichte Trübung in der Lösung. Anschließend werden 10 ml einer 5%igen Natriumchloridlösung ebenfalls unter ständigen Rühren zugegeben. Dabei entsteht ein flockiger Niederschlag, der sich über Nacht am Boden des Becherglases absetzt. Dieser Niederschlag enthält den gesamten Farbstoff und wird durch Zentrifugieren abgetrennt und anschließend dreimal mit 250 ml einer 0,5%igen NaCI-Lösung gewaschen. Im nächsten Schritt wird der Niederschlag mit 200 ml Ethanol gewaschen, um den an der Oberfläche adsorbierten Lumineszenzfarbstoff vollständig auszuwaschen. Der Ethanol wird durch Zentrifugieren vom Niederschlag abgetrennt. Anschließend erfolgt ein letzter Waschschritt in einer 0,05%igen NaCI-Lösung. Der Niederschlag, der aus den Nanopartikeln besteht, wird abgetrennt und in 50 ml H₂O aufgenommen.

### Beispiel 2

### Herstellung von phosphoreszierenden Nanopartikeln aus Polyacrylnitril und [Ruthenium(II)-tris-1,10 phenanthrolin]²⁺

1 g n-Polyacrylnitril wird zusammen mit 10 mg Ruthenium(II))-tris-1,10 phenanthrolin-hexafluorophosphat in 100 ml Dimethylformamid gelöst und in ein 1 l Becherglas gefüllt. Zu dieser Lösung werden 400 ml H₂O unter ständigem Rühren langsam zugetropft. Es entsteht eine leichte Trübung in der Lösung. Anschließend werden 10 ml einer 5%igen Natriumchloridlösung ebenfalls unter ständigen Rühren zugegeben. Dabei entsteht ein Niederschlag, der sich über Nacht am Boden des Becherglases absetzt. Dieser Niederschlag enthält ca. 90% des eingesetzten Farbstoffs und wird durch Zentrifugieren abgetrennt und anschließend dreimal mit 250 ml einer 0,5%igen NaCI-Lösung gewaschen. Im nächsten Schritt wird der Niederschlag mit 200 ml Ethanol gewaschen, um den an der Oberfläche adsorbierten Lumineszenzfarbstoff vollständig auszuwaschen. Der Ethanol wird durch Zentrifugieren vom Niederschlag abgetrennt. Anschließend erfolgt ein letzter Waschschritt in einer 0,05%igen NaCI-Lösung. Der Niederschlag (Nanopartikel) wird abgetrennt und in 50 ml H₂O aufgenommen.

### Beispiel 3

### Carboxylierung der Oberfläche der lumineszierenden Nanopartikel

10 ml der Partikelsuspension aus den Beispielen 1 oder 2 mit einem Feststoffgehalt von 200 mg Polyacrylnitril werden in 50 ml einer 5%igen NaOH-Lösung aufgenommen. Die Partikel fallen aus. Die Suspension wird für 45 Minuten unter intensiven Rühren auf 75 °C erhitzt. intensiver Geruch nach Ammoniak zeigt die Verseifung der an den Oberflächen befindlichen Nitrilgruppen an. Nach Aufklaren der trüben Lösung wird die Natronlauge durch Zugabe von HCI neutralisiert und auf pH 3 eingestellt. Dabei fallen die an der Oberläche carboxylierten Partikel wiederum als Niederschlag aus und können abzentrifugiert werden. Abschließend werden sie in in 50 ml Puffer pH 3 gewaschen, abzentrifugiert und in 10 ml destilliertem Wasser aufgenommen.

Auf analoge Weise kann die Verseifung auch in 8% NaOH bei 25°C für 24 h erfolgen.

### Beispiel 4

### Nanopartikel bestehend aus einem Copolymer aus 90% Polyacrylnitril und 10% Polyacrylsäure und [Ruthenium (II)-tris-4,7-diphenyl-1,10-phenantrhrolin]²⁺

2 g eines selbstsysnthetisierten Acrylnitril-Acrylsäure 10:1 Copolymers und 40 mg [Ruthenium (II)-tris-4,7-diphenyl-1,10-phenanthrolin]²⁺ als Trimethylsilylpropansulfonat (Ru(dphphen)₃TMS₂) werden in 400 g DMF gelöst. Unter Rühren wird 1 l 10⁻³ N NaOH zugetropft und mit Wasser auf 2 I aufgefüllt. Die klare Suspension wird mit 0,1 N HCI auf pH 3 gebracht und der Niederschlag durch Zentrifugation abgetrennt. Das Zentrifugat wird 3 mal mit je 1,8 I Wasser gewaschen und in 200 ml 50 mM Na₂HPO₄ mittels Ultraschall resuspendiert. Die klare Suspension wird 20 min auf ca 80°C erwärmt und nach dem Abkühlen erneut durch HCI Zugabe auf pH 3 gebracht, abzentrifugiert und in 200 ml 50 mM Na₂HPO₄ mittels Ultraschall resuspendiert.

### Beispiel 5

### Nanopartikel bestehend aus einem Copolymer aus 95% Polyacrylnitril und 5% Polyacrylsäure und [Ru(dph phen)₃]²⁺

2 g Acrylnitril-Acrylsäure 20:1 Copolymer und 40 mg Ru(dphphen)₃TMS₂ werden in 400 g DMF gelöst. Unter Rühren wird 1l 10⁻³ N NaOH zugetropft und mit Wasser auf 2 l aufgefüllt. Die klare Suspension wird mit 0,1 N HCI auf pH 3 gebracht und der Niederschlag durch Zentrifugation abgetrennt. Das Zentrifugat wird 3 mal mit je 1,8 l Wasser gewaschen und in 200 ml 50 mM Na₂HPO₄ mittels Ultraschall resuspendiert. Die klare Suspension wird 20 min auf ca 80°C erwärmt und nach dem Abkühlen erneut durch HCI Zugabe auf pH 3 gebracht, abzentrifugiert und in 200 ml 50 mM Na₂HPO₄ mittels Ultraschall resuspendiert.

### Beispiel 6

### Nanopartikel bestehend aus einem Copolymer aus 99,5% Polyacrylnitril und 0,5% Polyacrylamin und [Ru(dph phen)₃]²⁺

0.5 g Acrylnitril - 3-Aminopropylacrylamid - 200:1 Copolymer und 10 mg Ru(dphphen)₃TMS₂ werden in 100 g DMF gelöst. Unter Rühren wird 0.5 l 10⁻³ N HCl zugetropft und mit Wasser auf 1 l aufgefüllt. Die klare Suspension wird mit 0,1 N NaOH auf pH 9 gebracht und der Niederschlag durch Zentrifugation abgetrennt. Das Zentrifugat wird 3 mal mit je 1 l Wasser gewaschen und in 50 ml Wasser mittels Ultraschall resuspendiert. Die Suspension wird 20 min auf ca 80°C erwärmt und nach dem Abkühlen 2 mal mit Wasser gewaschen und resuspendiert.

### Beispiel 7

### Nanopartikel bestehend aus einem Copolymer aus 90% Polyacrylnitril und 5% Polyacrylsäure und 5% Polyethytenglycolmonoethyletheraerylat und [Ru(dph phen)₃]²⁺

0.5 g Acrylnitril-Acrylsäure- Polyethylenglycolmonoethyletheracrylat 20:1:1 Copolymer und 5 mg Ru(dphphen)₃TMS₂ werden in 200 g DMF gelöst. Unter Rühren wird 1 l 10⁻³ N NaOH zugetropft. Die klare Suspension wird mit 0.1 N HCI auf pH 3 gebracht und der Niederschlag durch Zentrifugation abgetrennt. Das Zentrifugat wird 3 mal mit je 1 l Wasser gewaschen und in 1 l100 mM Na₂HPO₄ mittels Ultraschall resuspendiert. Die klare Suspension wird durch HCI Zugabe auf pH 3 gebracht, abzentrifugiert und in 200 ml 100 mM Na₂HPO₄ mittels Ultraschall resuspendiert. Die klare Suspension wird 20 min auf ca 80°C erwärmt und nach dem Abkühlen erneut durch HCI Zugabe auf pH 3 gebracht, abzentrifugiert und in 200 ml 50 mM Na₂HPO₄ mittels Ultraschall resuspendiert.

### Beispiel 8

### Nanopartikel bestehend aus einem Copolymer aus 85% Polyacrylnitril, 5% Polyacrylsäure und 10 % Polysulfoacrylat und [Ru(dph phen)₃]²⁺

0.5 g Acrylnitril-Acrylsäure-Sulfopropylacrylat 20:1:2 Copolymer und 50 mg Ru(dphphen)₃Cl₂ werden in 100 g DMF gelöst. Unter Rühren wird 0.5 l 10⁻³ N NaOH zugetropft. Die klare Suspension wird mit 0.1 N HCI auf pH 3 gebracht und der Niederschlag durch Zentrifugation abgetrennt. Das Zentrifugat wird 3 mal mit je 1 l Wasser gewaschen und in 100 ml 50 mM Na₂HPO₄ mittels Ultraschall resuspendiert. Die klare Suspension wird 20 min auf ca 80°C erwärmt und nach dem Abkühlen erneut durch HCI Zugabe auf pH 3 gebracht abzentrifugiert und in 100 ml 50 mM Na₂HPO₄ mittels Ultraschall resuspendiert.

### Beispiel 9

### Charakterisierung von lumineszierenden Partikeln auf Basis von Polyacrylnitril oder Polyacrylnitril-Copolymeren

Die aufgelisteten Partikel mit einem mittleren Durchmesser von 20 bis 100 nm und dem Lumineszenzfarbstoff Ruthenium(II)-tris-4,7-diphenyl-1,10-phenanthrolin wurden bei 20°C in einem 20 mM Phosphatpuffer (pH 7) vermessen. Die Nanopartikel waren in einer Probe dispergiert. Die Ergebnisse sind in der folgenden Tabelle I dargestellt.

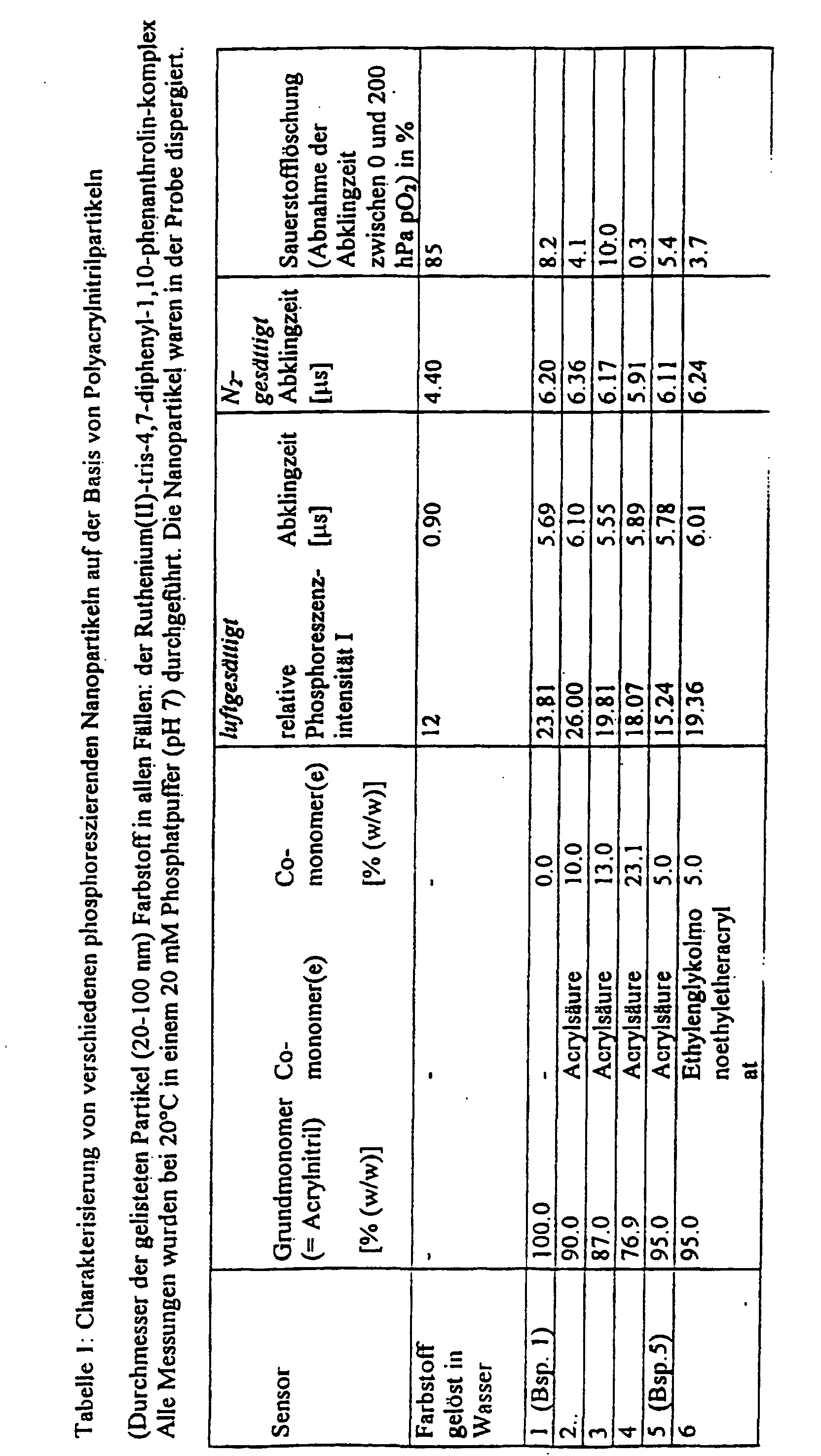

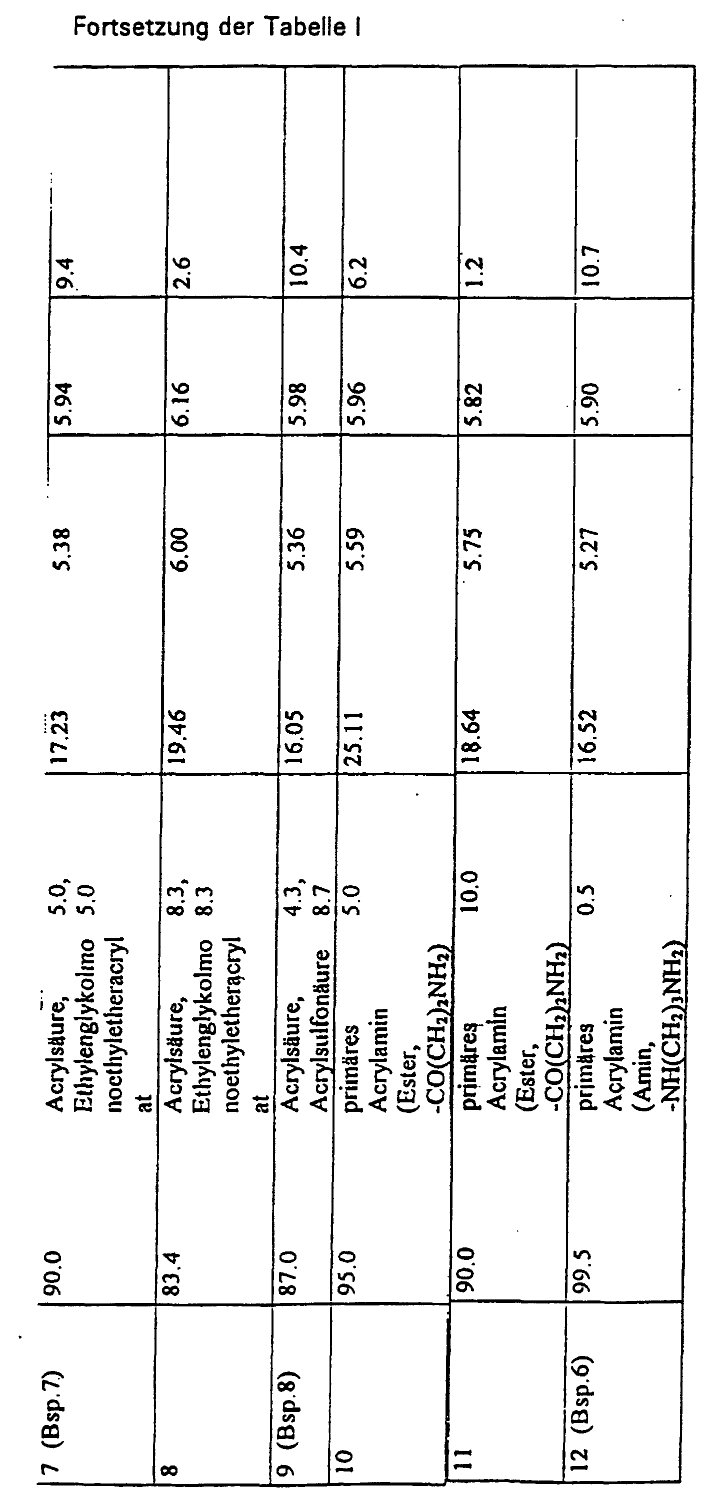

## Patentansprüche

1. Lumineszierende Mikro- und Nanopartikel,
**dadurch gekennzeichnet,**
**dass** sie lumineszierende Substanzen mit langen Lumineszenzabklingzeiten enthalten und wobei die lumineszierenden Substanzen gegenüber chemischen, biochemischen und gasförmigen Parametern der Umgebung im Wesentlichen abgeschirmt sind.

2. Partikel nach Anspruch 1,
**dadurch gekennzeichnet,**
**dass** eine oder mehrere Lumineszenzeigenschaften der lumineszierenden Substanzen, insbesondere ausgewählt aus Quantenausbeute, spektraler Charakteristik, Lumineszenzabklingzeit und Anisotropie von der jeweiligen Umgebung im Wesentlichen unabhängig sind.

3. Partikel nach Anspruch 1 oder 2,
**dadurch gekennzeichnet,**
**dass** es sich bei den lumineszierenden Substanzen um Metall-Liganden-Komplexe von Ruthenium(II), Osmium(II) Rhenium(I), tridium(III) Platin(II) und Palladium(II)als Zentralatom handelt.

4. Partikel nach Anspruche 3,
**dadurch gekennzeichnet,**
**dass** es sich bei den lumineszierenden Substanzen um Komplexe mit 2 oder 3-zähnigen Polypyridylliganden wie 2,2'-Bipyridin, Bipyrazin, Phenanthrolin, Terpyridyl oder deren Abkömmlingen als Liganden handelt.

5. Partikel nach einem der Ansprüche 3 - 4,
**dadurch gekennzeichnet,**
**dass** es sich bei den lumineszierenden Verbindungen um die Triskomplexe von Ruthenium(II) mit 2,2 '-Bipyridyl, 1,10-Phenanthrolin, 4,4-Diphenyl-2,2'-bipyr'sdyl und 4,7-Diphenyl-1,10-phenanthrolin als Liganden handelt.

6. Partikel nach Anspruche 1 oder 2,
**dadurch gekennzeichnet,**
**dass** es sich bei den lumineszierenden Substanzen um Carbonylkomplexe von Re(I) mit zusätzlichen Diiminliganden wie Abkömmlingen von 2,2 '-Bipyridyl und 1,10-Phenanthrolin handelt.

7. Partikel nach Anspruch 1 oder 2,
**dadurch gekennzeichnet,**
**dass** es sich bei den lumineszierenden Verbindungen um Porphyrinkomplexe von Pt(II) sowie Pd(II) als Zentralatom handelt.

8. Partikel nach einem der Ansprüche 1 - 7,
**dadurch gekennzeichnet,**
**dass** sie ein organisches Polymer enthalten, das sich durch geringe Wasseraufnahme oder/und minimale Gaspermeabilität auszeichnet.

9. Partikel nach Anspruch 8,
**dadurch gekennzeichnet,**
**dass** sie ein organisches Polymer aus der Gruppe der Polyacrylnitrile, Poly(meth)acrylcopolymere, Polyvinylchloride oder Polyvinylidenchloride und Copolymere davon enthalten.

10. Partikel nach Anspruch 9,
**dadurch gekennzeichnet,**
**dass** sie Polyacrylnitril oder Polyacrylnitril-Copolymere, insbesondere Copolymere mit Acrylsäure, Acrylaminen oder/und Acrylestern enthalten.

11. Partikel nach einem der Ansprüche 1 - 7,
**dadurch gekennzeichnet,**
**dass** sie ein Glas enthalten, das im Wesentlichen frei von Mikroporen ist.

12. Partikel nach Anspruch 11,
**dadurch gekennzeichnet,**
**dass** sie ein Glas enthalten, das nach einem Sol-Gel-Verfahren hergestellt worden ist.

13. Partikel nach Anspruch 11 oder 12,
**dadurch gekennzeichnet,**
**dass** sie ein Sol-Gel Glas enthalten, das aus Silizium-, Titan-, Zirkonium- oder/und Zinn-tetraalkoholaten hergestellt worden ist.

14. Partikel nach einem der Ansprüche 1 - 13,
**dadurch gekennzeichnet,**
**dass** ihre Oberfläche durch reaktive Gruppen wie Amino-, Epoxy-, Hydroxy-, Thiol- oder/und Carboxylgruppen modifiziert ist, weiche die kovalente Kopplung von Lumineszenzindikatoren oder/und Biomolekülen ermöglichen.

15. Partikel nach Anspruch 14,
**dadurch gekennzeichnet,**
**dass** sie an ihrer Oberfläche kovalent gekoppelt Lumineszenzindikatoren oder/und Biomoleküle enthalten.

16. Verfahren zur Herstellung von lumineszierenden Mikro- und Nanopartikeln nach einem der Ansprüche 8 - 10, wobei die Partikel aus einer Polymerlösung, in welcher die lumineszierende Verbindung gelöst vorliegt, durch Zutropfen einer Flüssigkeit ausgefällt werden, wobei die Flüssigkeit mit dem Polymerlösungsmittel mischbar ist, jedoch eine Verringerung der Löslichkeit des Polymers bewirkt.

17. Verfahren nach Anspruch 1,6, wobei die Partikel aus einer Lösung bestehend aus Dimethylformamid und Polyacrylnitril oder Polyacrylnitril-Copolymer, in welcher die lumineszierende Verbindung gelöst vorliegt, durch Zutropfen von Wasser oder einer wässrigen Lösung ausgefällt werden.

18. Verfahren nach Anspruch 16 oder 17, wobei der Durchmesser der Partikel durch Variation des Polymergehaltes der Lösung eingestellt wird.

19. Verfahren zur Herstellung von lumineszierenden Mikro- und Nanopartikeln nach einem der Ansprüche 8 - 10, wobei die lumineszierende Verbindung in bereits vorgefertigte Partikel aus einem Lösungsmittel(gemisch) durch Diffusion eingebaut wird.

20. Verfahren zur Herstellung von lumineszierenden Mikro- und Nanopartikeln nach einem der Ansprüche 8 - 10, wobei die Partikel durch Versprühen einer Polymerlösung, in welcher die lumineszieremde Verbindung gelöst vorliegt und Verdampfung des Lösungsmittels entstehen.

21. Verfahren nach Anspruch 20 wobei der Durchmesser der Partikel durch Variation des Polymergehaltes der Sprühlösung eingestellt wird.

22. Verfahren zur Herstellung von lumineszierenden Mikropartikeln nach einem der Ansprüche 11 - 13, wobei die lumineszierende Verbindung in verdichtete monolithische Sol-Gel Gläser eingebaut wird, welche anschließend gemahlen und nach Größe fraktioniert werden.

23. Verwendung der lumineszierenden Mikro- und Nanopartikel nach einem der Ansprüche 1 - 14 zur Markierung und lumineszenzoptischen Detektion von Biomolekülen aus der Gruppe der Toxine, Hormone, Hormonrezeptoren, Peptide, Proteine, Lektine, Oligonukleotide, Nukleinsäuren, Antikörper, Antigene, Viren und Bakterien.

24. Verwendung der lumineszierenden Mikro- und Nanopartikel nach einem der Ansprüchen 1- 14 als Standards zur Referenzierung von Fluoreszenzintensitätssignalen in fluorimetrischen Assays.

25. Verwendung nach Anspruch 23, wobei durch die Zugabe des Standards zur Probe die Intensitätsinformation in ein Phasensignal oder/und einen zeitabhängigen Parameter konvertiert wird.

26. Verwendung der lumineszierenden Mikro- und Nanopartikel nach einem der Ansprüche 1 - 14 zur Referenzierung des Lumineszenzintensitätssignals von optischen Lumineszenzsensoren, wobei die Partikel mit einem Lumineszenzindikator in einer festen Phase gemeinsam immobilisiert werden.

27. Verfahren zur lumineszenzoptischen Bestimmung eines biochemischen oder chemischen Parameters unter Verwendung zweier verschiedener Lumineszenzfarbstoffe, die unterschiedliche Abklingzeiten aufweisen und das Zeit- oder Phasenverhalten der sich ergebenden Lumineszenzantwort zur Bildung einer Referenzgröße für die Bestimmung des Parameters verwendet wird, wobei der erste Lumineszenzfarbstoff zumindest in der Lumineszenzinterisität auf den Parameter anspricht und der zweite zumindest in der Lumineszenzintensität und der Lumineszenzabklingzeit nicht auf den Parameter anspricht,
**dadurch gekennzeichnet,**
**dass** der zweite Lumineszenzfarbstoff in Form von Partikeln nach einem der Ansprüche 1 - 15 eingesetzt wird.

## Claims

1. Luminescent micro- or nanoparticles,
**characterized in that**
they contain luminescent substances having long luminescence lifetimes and said luminescent substances are essentially shielded from ambient chemical, biochemical and gaseous parameters.

2. Particles according to Claim 1,
**characterized in that**
one or more luminescence properties of said luminescent substances, which are in particular selected from the group consisting of quantum yield, spectral characteristics, luminescence lifetime and anisotropy, are essentially independent of the particular environment.

3. Particles according to Claim 1 or 2,
**characterized in that**
the luminescent substances are metal/ligand complexes of ruthenium(II), osmium(II) rhenium(I), iridium(III) platinum(II) and palladium(II) as central atoms.

4. Particles according to Claim 3,
**characterized in that**
the luminescent substances are complexes with 2-or 3-dentate polypyridyl ligands such as 2,2'-bipyridine, bipyrazine, phenanthroline, terpyridyl or derivatives thereof as ligands.

5. Particles according to either of Claims 3 - 4,
**characterized in that**
the luminescent compounds are the tris complexes of ruthenium (II) with 2,2'-bipyridyl, 1,10-phenanthroline, 4,4-diphenyl-2,2'-bipyridyl and 4,7-diphenyl-1,10-phenanthroline as ligands.

6. Particles according to Claim 1 or 2,
**characterized in that**
the luminescent substances are carbonyl complexes of Re(I) with additional diimine ligands such as derivatives of 2,2'-bipyridyl and 1,10-phenanthroline.

7. Particles according to Claim 1 or 2,
**characterized in that**
the luminescent compounds are porphyrin complexes of Pt(II) and Pd(II) as central atoms.

8. Particles according to any of Claims 1-7,
**characterized in that**
they contain an organic polymer which distinguishes itself by low absorption of water or/and minimum gas permeability.

9. Particles according to Claim 8,
**characterized in that**
they contain an organic polymer from the group consisting of polyacrylonitrile, poly(meth)acrylic copolymers, polyvinyl chlorides or polyvinylidene chlorides and copolymers thereof.

10. Particles according to Claim 9,
**characterized in that**
they contain polyacrylonitrile or polyacrylonitrile copolymers, in particular copolymers with acrylic acid, acrylic amines or/and acrylic esters.

11. Particles according to any of Claims 1-7,
**characterized in that**
they contain a glass which is essentially free of micropores.

12. Particles according to Claim 11,
**characterized in that**
they contain a glass which has been produced according to a sol/gel process.

13. Particles according to Claim 11 or 12,
**characterized in that**
they contain a sol/gel glass which has been prepared from silicon, titanium, zirconium or/and tin tetraalcoholates.

14. Particles according to any of Claims 1 - 13,
**characterized in that**
their surface has been modified by reactive groups such as amino, epoxy, hydroxyl, thiol or/and carboxyl groups which make possible the covalent coupling of luminescent indicators or/and biomolecules.

15. Particles according to Claim 14,
**characterized in that**
they contain luminescent indicators or/and biomolecules covalently coupled to their surface.

16. Method for preparing luminescent micro- and nanoparticles according to any of Claims 8 - 10, wherein the particles are precipitated from a polymer solution in which the luminescent compound is present in soluble form by adding a liquid dropwise, with the liquid being miscible with the polymer solvent but causing a reduction in the solubility of the polymer.

17. Method according to Claim 16, wherein the particles are precipitated from a solution comprising dimethylformamide and polyacrylonitrile or polyacrylonitrile copolymer, in which the luminescent compound is present in soluble form, by adding water or an aqueous solution dropwise.

18. Method according to Claim 16 or 17, wherein the particle diameter is adjusted by varying the polymer content of the solution.

19. Method for preparing luminescent micro- and nanoparticles according to any of Claims 8-10, wherein the luminescent compound is incorporated by diffusion from a solvent (mixture) into already prefabricated particles.

20. Method for preparing luminescent micro- and nanoparticles according to any of Claims 8-10, wherein the particles are formed by spraying a polymer solution in which the luminescent compound is present in soluble form and evaporation of the solvent.

21. Method according to Claim 20, wherein the particle diameter is adjusted by varying the polymer content of the spray solution.

22. Method for preparing luminescent microparticles according to any of Claims 11-13, wherein the luminescent compound is incorporated into compressed monolithic sol/gel glasses which are subsequently ground and fractionated according to size.

23. Use of the luminescent micro- and nanoparticles according to any of Claims 1 - 14 for labelling and luminometric detection of biomolecules from the group consisting of toxins, hormones, hormone receptors, peptides, proteins, lectins, oligonucleotides, nucleic acids, antibodies, antigens, viruses and bacteria.

24. Use of the luminescent micro- and nanoparticles according to any of Claims 1 - 14 as reference standards of fluorescence intensity signals in fluorimetric assays.

25. Use according to Claim 23, wherein addition of the standard to the sample converts the intensity information into a phase signal or/and a time-dependent parameter.

26. Use of the luminescent micro- and nanoparticles according to any of Claims 1 - 14 for referencing the luminescence intensity signal of optical luminescence sensors, wherein the particles are immobilized to a solid phase together with a luminescent indicator.

27. Method for luminometric determination of a biochemical or chemical parameter using two different luminescent dyes which have different lifetimes and the time or phase characteristics of the resulting luminescent response are used for generating a reference parameter for determination of said parameter, with the first luminescent dye corresponding to said parameter at least with respect to luminescence intensity and the second one not corresponding to said parameter at least with respect to luminescence intensity and luminescence lifetime,
**characterized in that**
the second luminescent dye is used in the form of particles according to any of Claims 1-15.

## Revendications

1. Microparticules et nanoparticules luminescentes, **caractérisées en ce qu'**elles comprennent des substances luminescentes avec des temps d'établissement de luminescence longs et dans lesquelles les substances luminescentes sont essentiellement protégées contre des paramètres chimiques, biochimiques et gazeux de l'environnement.

2. Particules selon la revendication 1, **caractérisées en ce que** une ou plusieurs propriétés de luminescence des substances luminescentes, choisies en particulier parmi le rendement quantique de conversion, la caractéristique spectrale, le temps d'établissement de luminescence et l'anisotropie sont essentiellement indépendantes de chacun des environnements.

3. Particules selon la revendication 1 ou 2, **caractérisées en ce que** l'atome central dans les substances luminescentes est formé de complexes métal-ligands de ruthénium(II), d'osmium(II), de rhénium(I), d'iridium(III), de platine(II) et de palladium(II).

4. Particules selon la revendication 3, **caractérisées en ce que** les ligands dans les substances luminescentes sont des complexes avec des ligands polypyridyle bidentés ou tridentés, comme 2,2'-bipyridine, bipyrazine, phénanthroline, terpyridyle ou leurs dérivés.

5. Particules selon les revendications 3 à 4, **caractérisées en ce que** les ligands dans les composés luminescents sont les tris-complexes de ruthénium(II) avec 2,2'-bipyridyle, 1,10-phénanthroline, 4,4-diphényle-2,2'-bipyridyle et 4,7-diphényle- 1,10-phénanthroline.

6. Particules selon la revendication 1 ou 2, **caractérisées en ce que** les substances luminescentes sont des complexes carbonyle de Rc(I) avec des ligands diimine comme des dérivés de 2,2'-bipyridile et de 1,10-phénanthroline.

7. Particules selon la revendication 1 ou 2, **caractérisées en ce que** l'atome central dans les composés luminescents est formé de complexes de porphyrine de Pt(II) ainsi que de Pd(III).

8. Particules selon l'une des revendications 1 à 7, **caractérisées en ce qu'**elles comprennent un polymère organique qui se distingue par une faible absorption d'eau ou/et une perméabilité au gaz minimale.

9. Particules selon la revendication 8, **caractérisées en ce qu'**elles comprennent un polymère organique du groupe des polyacrylonitrile, des copolymères poly(méth)acryliques, des chlorures de polyvinyle ou des chlorures de polyvinylidènes et des copolymères de ceux-ci.

10. Particules selon la revendication 9, **caractérisées en ce qu'**elles comprennent du polyacrylonitrile ou des copolymères de polyacrylonitrile, en particulier des copolymères avec de l'acide acrylique, des amines acryliques ou/et des esters acryliques.

11. Particules selon l'une des revendications 1 à 7, **caractérisées en ce qu'**elles comprennent un verre essentiellement exempt de micropores.

12. Particules selon la revendication 11, **caractérisées en ce qu'**elles comprennent un verre qui est produit selon un procédé sol-gel.

13. Particules selon la revendication 11 ou 12, **caractérisées en ce qu'**elles comprennent un verre sol-gel, qui est produit à partir de tétraalcoolats de silicium, de titane, de zirconium ou/et d'étain.

14. Particules selon les revendications 1 à 13, **caractérisées en ce que** leur surface est modifiée par des groupes réactifs comme des groupes amino, des groupes époxy, des groupes hydroxy, des groupes thiol ou/et des groupes carboxyle, qui permettent le couplage covalent d'indicateurs de luminescence ou/et de biomolécules.

15. Particules selon la revendication 14, **caractérisées en ce qu'**elles comprennent à leur surface des indicateurs de luminescence ou/et des biomolécules couplés de manière covalente.

16. Procédé pour la fabrication de microparticules et de nanoparticules selon l'une des revendications 8 à 10, dans lequel les particules d'une solution polymère, dans laquelle le composé luminescent est présent sous forme dissoute, sont précipitées par l'ajout goutte à goutte d'un liquide, le liquide pouvant être miscible avec le solvant polymère mais produisant cependant une diminution de la solubilité du polymère.

17. Procédé selon la revendication 16, dans lequel les particules d'une solution se composant de diméthylformamide et de polyacrylonitrile ou d'un copolymère de polyacrylonitrile, dans laquelle le composé luminescent est présent sous forme dissoute, sont précipitées par ajout goutte à goutte d'eau ou d'une solution aqueuse.

18. Procédé selon la revendication 16 ou 17, dans lequel le diamètre des particules est établi par la variation de la teneur en polymère de la solution.

19. Procédé pour la fabrication de microparticules et de nanoparticules selon l'une des revendications 8 à 10, dans lequel le composé luminescent est introduit par diffusion dans des particules déjà préformées d'un (mélange de) solvant.

20. Procédé pour la fabrication de microparticules et de nanoparticules selon l'une des revendications 8 à 10, dans lequel les particules proviennent de la pulvérisation d'une solution polymère, dans laquelle le composé luminescent est présent sous forme dissoute, et de l'évaporation du solvant.

21. Procédé selon la revendication 20, sachant que le diamètre des particules est établi par la variation de la teneur en polymère de la solution de pulvérisation.

22. Procédé pour la fabrication de microparticules luminescentes selon l'une des revendications 11 à 13, dans lequel le composé luminescent est introduit dans des verres sol-gel comprimés monolithiques, qui sont par la suite moulus et fractionnés selon la taille.

23. Utilisation des microparticules et nanoparticules luminescentes selon l'une des revendications 1 à 14 pour le marquage et la détection par luminescence optique de biomolécules du groupe des toxines, des hormones, des récepteurs d'hormones, des peptides, des protéines, des lectines, des oligonucléotides, des acides nucléiques, des anticorps, des antigènes, des virus et des bactéries.

24. Utilisation des microparticules et des nanoparticules selon l'une des revendications 1 à 14 comme normes pour référencer des signaux d'intensité de fluorescence dans des analyses fluorimétriques.

25. Utilisation selon la revendication 23, sachant que l'information d'intensité est convertie en un signal de phase ou/et un paramètre dépendant du temps par adjonction de la norme dans l'échantillon.

26. Utilisation des microparticules et des nanoparticules selon l'une des revendications 1 à 14 pour référencer le signal d'intensité de luminescence de capteurs optiques de luminescence, dans laquelle les particules sont immobilisées conjointement avec un indicateur de luminescence dans une phase solide.

27. Procédé pour la définition par luminescence optique d'un paramètre biochimique ou chimique par utilisation de deux colorants différents de luminescence qui comportent des temps d'établissement différents et dans lequel le comportement dans le temps et la caractéristique de phase de la réponse de luminescence qui en résulte sont utilisés pour la constitution d'une grandeur de référence pour la définition du paramètre, dans lequel le premier colorant de luminescence correspond, au moins dans l'intensité de luminescence, au paramètre, et que le deuxième ne correspond pas, au moins dans l'intensité de luminescence et le temps d'établissement de la luminescence, au paramètre, **caractérisé en ce que** le deuxième colorant de luminescence est mis en oeuvre sous la forme de particules selon l'une des revendications 1 à 15.
